# EUROPÄISCHE PATENTSCHRIFT

(11) **EP 0 920 248 B1**
(45) Veröffentlichungstag und Bekanntmachung des Hinweises auf die Patenterteilung: **20.03.2002**
(21) Anmeldenummer: 97938837.8
(22) Anmeldetag: 21.07.1997
(51) Int. Cl.: A01H 4/00

(54) **IN HYDROGELEN EINGEBETTETES BIOLOGISCHES MATERIAL, EIN VERFAHREN ZU DESSEN EINBETTUNG SOWIE DESSEN VERWENDUNG ALS KÜNSTLICHES SAATGUT**
BIOLOGICAL MATERIAL EMBEDDED IN HYDROGELS, A PROCESS FOR THE EMBEDDING THEREOF, AND ITS USE AS ARTIFICIAL SEED
SUBSTANCE BIOLOGIQUE INCORPOREE DANS DE L'HYDROGEL, PROCEDE D'INCORPORATION DE LADITE SUBSTANCE ET SON UTILISATION EN TANT QUE SEMENCE ARTIFICIELLE

(30) Priorität: 02.08.1996 DE 19631320
(43) Veröffentlichungstag der Anmeldung: 09.06.1999
(73) Patentinhaber: BAYER AG, 51368 Leverkusen (DE)
(72) Erfinder: LECHELT-KUNZE, Christa, D-50935 Köln (DE); SIMON, Joachim, D-40589 Düsseldorf (DE); ZITZMANN, Werner, D-51381 Leverkusen (DE); KALBE, Jochen, D-42799 Leichlingen (DE); MÜLLER, Hanns-Peter, D-51519 Odenthal (DE); KOCH, Rainhard, D-51065 Köln (DE)
(86) Internationale Anmeldenummer: EP9703906
(87) Internationale Veröffentlichungsnummer: WO9805197

(56) Entgegenhaltungen:
- EP-A- 0 107 141
- WO-A-96/35733
- DE-A- 3 312 578
- DE-A- 4 217 891

## Beschreibung

Die Erfindung betrifft ein vollständig biologisch abbaubares Hydrogel bestehend aus Polyesterpolyurethanpolyharnstoff sowie Polysacchariden und/oder deren Derivaten, welches teilungsfähiges pflanzliches Material enthält.

Weiterhin betrifft die Erfindung ein Verfahren zur Einbettung des biologischen Materials und zur Herstellung und Formgebung der Hydrogele aus wäßrigen Lösungen sowie die Verwendung des in Hydrogelen eingebetteten biologischen Materials als künstliches Saatgut.

Die Vermehrung von Pflanzen erfolgt geschlechtlich über Samen und ungeschlechtlich oder vegetativ über Meristeme der Pflanzen. Beide Vermehrungsformen haben große wirtschaftliche Bedeutung. Während die Aussaat durch natürliche Samen weitgehend maschinell erfolgt, beinhaltet die vegetative Vermehrung viel Handarbeit und ist daher zeitaufwendiger, arbeitsintensiver und damit teurer als die Vermehrung durch Samen.

Vegetativ vermehrt werden Pflanzenarten, -sorten, -kultivare und -linien, bei denen es auf den Erhalt einer bestimmten genetischen Konstitution ankommt, (z.B. klonale Vermehrung von Elitepflanzen). Vegetativ vermehrt werden weiterhin Pflanzen, die nur nach langer Vegetationszeit Samen bilden, die nur wenig Samen bilden, oder deren Samen in der Keimfähigkeit beeinträchtigt sind.

Zur Vereinfachung der vegetativen Pflanzenvermehrung sind neben der Entwicklung von automatisierbaren Verfahren zur Massenanzucht, auch geeignete Stoffe und Verfahren zur Ummantelung des fragilen Materials wünschenswert, die weitgehend die Funktion einer Samenhülle übernehmen.

Bei einigen Pflanzenarten ist es gelungen miniaturisierte, teilungs- und regenerationsfähige Pflanzen(gewebe) in Massenanzuchtverfahren zu produzieren (z.B. WO95/19102, US 5 294 549, US 5 334 530). Diese Pflanzenteile sind ohne mechanischen Schutz und/oder Schutz vor Austrocknung nur begrenzt transport- und lagerfähig. Es ist daher wünschenswert Pflanzenteile als diskrete Einheiten so zu verkapseln, oder zu umhüllen, daß sie lager- und/oder transportfähig, ausreichend dosierbar sind und sich ähnlich wie natürliche, pflanzliche Samen verwenden lassen.

In DE 2 103 873, EP 141 374, EP 107 141, US 4 562 663, WO 8502972, US 4 779 376, WO 9207457 wird die Einbettung von Pflanzenmaterial in Hydrogele beschrieben, die aus ionisch vernetzbaren Polysacchariden wie z.B. Alginat, Gelatine, Carageenan oder locust bean gum" hergestellt wurden.

Die nach dem Stand der Technik bisher genannten Materialien, Kombinationen der Materialien und Verfahren sind bisher noch nicht zufriedenstellend, da sie teilweise den umhüllten Strukturen weder eine ausreichende mechanische Stabilität verleihen, noch das pflanzliche Gewebe vor einem zu schnellen oder zu weitreichenden Wasserverlust unter Gebrauchsbedingungen schützen. Dies gilt insbesondere für die genannten Polysaccharidderivate. Beim Austrocknen ist weiterhin ein starker Schrumpf der Materialien zu beobachten, der die Schutzfunktion der Hülle stark beeinträchtigen kann. Ein weiteres Problem speziell der bislang verwendeten Umhüllungen auf der Basis von Polysacchariden wie z.B. Alginsäuren, bzw. deren Salze, oder weiteren ionischen Polysaccharidderivaten besteht in der nicht ausreichenden Rehydratisierung nach einem Austrocknen. Diese Materialien können deshalb nur unter entsprechender Luftfeuchtigkeit aufbewahrt werden.

Nachträglich aufgebrachte Ummantelungen aus Fetten, Ölen, Wachsen oder nicht wasserlöslichen Polymeren zur Verzögerung der Dehydratisierung und mechanischer Stabilisierung, wie z.B. in US 4 562 663, WO 9217422, US 5 190 797 angegeben sind ebenfalls ungeeignet, wenn sie unter unphysiologisch hohen Temperaturen verarbeitet werden müssen, wenn sie die Benutzung organischer Lösungsmittel erfordern, oder wenn sie die Sauerstoffversorgung des eingeschlossenen biologischen Materials negativ beeinflussen.

Neben Hydrogelen auf Polysaccharidbasis werden auch Polyurethan (PU)-Hydrogele beschrieben In DE 3312578 und DE 4 217 891 wird zur Immobilisierung von teilungsfähigen Zellen die Verwendung von Polyurethanen beschrieben. PU-Hydrogele dienen in dieser Anwendung als Trägermaterial von Zellen und Biokatalysatoren in wäßrigen Suspensionen, wobei die für diesen Zweck beschriebenen PU-Hydrogele jedoch nicht biologisch abbaubar sind.

Es ist Aufgabe der vorliegenden Erfindung, eine Verkapselung/Verpackung von teilungsfähigem, biologischem Material zum Zweck des Schutzes des Materials bei Lagerung, Transport und Handhabung zur Verfügung zu stellen, welches die Austrocknung stark verzögert, formstabil ist, nach partieller Austrockung in ausreichendem Maßen wiederanquellbar ist, biologisch abbaubar ist und leicht herzustellen ist.

Ebenso soll die Beigabe von Zusätzen wie Nährstoffen oder Wirk- und Schutzstoffen möglich sein.

Die Handhabung des benötigten Materials muß unter sterilen Bedingungen erfolgen können und ohne toxische Lösungsmittel oder physiologisch nicht akzeptable Bedingungen auskommen.

Die Lösung der oben beschriebenen Aufgaben wird überraschenderweise durch die Verwendung von biologisch vollständig abbaubaren Polyesterpolyurethanpolyharnstoffen in Kombination mit Polysacchariden oder Polysaccharidderivaten erreicht, die als Dispersion in Wasser oder wäßrigen Lösungen eingesetzt werden können.

Es wurde überraschenderweise gefunden, daß Polyesterpolyurethanpolyharnstoffe sich für die Umhüllung biologischen Materials eignen und durch Kombination mit biologisch abbaubaren Polysacchariden oder deren Derivaten zur erfindungsgemäßen Einbettung im Sinne der beschriebenen Aufgabe der Erfindung verwendet werden können.

Gegenstand der Erfindung sind biologisch abbaubare Hydrogele enthaltend wenigstens
A) einen Polyesterpolyurethanpolyharnstoff, sowie
B) Polysaccharide und/oder Polysaccharidderivate, und
C) biologisches Material, bevorzugt teilungsfähiges pflanzliches Material, insbesondere Pflanzenzellen, Kallusgewebe, Protoplasten, Pflanzengewebe oder Pflanzenorgane, wie z.B. Adventivsprossen, Mikroknollen, Achselknospen, Apikalknospen, Sprößlinge, sowie zygotische oder somatische Embryonen oder Protocorm-Analoge.

Das Pflanzenmaterial kann aus den folgenden Pflanzen stammen: nahrungs- und rohstoffliefernde Pflanzen, z.B. Getreide (z.B. Reis, Mais, Weizen, Gerste, Roggen, Hirse), Kartoffel, Leguminosen (z.B. Luzerne und Sojabohnen), Raps, Sonnenblumen, Ölpalme, Zuckerrohr, Zuckerrübe, Sisal, Baumwolle, Miscanthus und Tabak; Gemüse und Gewürzpflanzen (z.B. Tomate, Kohlarten, Salat, Karotte, Aubergine, Melone, Gurke, Spargel, Zwiebeln, Petersilie, Ingwer); Heilpflanzen wie Ginseng, Tollkirsche, Digitalis; Obst (z.B. Äpfel, Birnen, Kirschen, Weintrauben, Erdbeeren, Citrus, Mango, Papaya, Banane, Nüsse); Tee-, Kakao-, Kaffeesträucher; Forstpflanzen z.B. Coniferen wie Fichte, Tanne, Kiefer, Lärche, Laubbäume z.B. Pappeln, Buchen, Eichen; Zierpflanzen z.B. Rose, Chrysantheme, Lilie, Amaryllis, Orchidee, Geranie, Begonie, Nelke, Anthurium.

Bevorzugt eingesetzt werden können desweiteren solche teilungsfähigen biologischen Materialien, die besonders bevorzugt aus transgenen Pflanzen stammen, bei welchen durch die Art der gentechnischen Veränderung, z.B. durch Samen- oder knollenspezifische Expression der Produkte, eine Vermehrung über Samen oder über vegetative Organe nicht mehr oder nur unter Schwierigkeiten möglich ist.

Mit dem Begriff "Einbettung" werden im folgenden alle möglichen Prozesse der Verkapselung, Umhüllung, Beschichtung, Verpackung etc. des erfindungsgemäßen biologischen Materials beschrieben.

Die biologische Abbaubarkeit von Materialien orientiert sich an den Anforderungen unter Standard-Bedingungen (siehe Beispiel 6).

Erfindungsgemäß können die Polyesterpolyurethanpolyharnstoffe in Mischungen mit ionischen oder neutralen biologisch abbaubaren Polysacchariden und deren Derivaten in einem ein- oder mehrstufigen Prozeß eingesetzt werden, um Formkörper, z.B. Kugeln, Fasern, Folien, Beschichtungen oder Ähnliches zu bilden.

Durch die Polysaccharide wird eine Wasser enthaltende Matrix (Hydrogel) gebildet, durch den Polyesterpolyurethanpolyharnstoff werden die mechanischen Eigenschaften des Hydrogels in überraschender Weise soweit verbessert, daß die Herstellung von einfachen Formkörpern z.B. Kugeln ermöglicht wird und der Wasserverlust des Hydrogels sowie des erfindungsgemäßen biologischen Materials kontrolliert werden kann.

Die erfindungsgemäß eingesetzten Polyesterpolyurethanpolyharnstoffes sind aus der DE 19 517 185 bekannt.

Zu ihrer Herstellung werden unter Einhaltung eines Äquivalentverhältnisses von Isocyanatgruppen zu gegenüber Isocyanatgruppen reaktionsfähigen Gruppen von 1:1 bis 2:1
a) eine Diisocyanatkomponente, bestehend aus
   a1) Hexamethylendiisocyanat oder
   a2) Gemischen aus Hexamethylendiisocyanat mit insgesamt bis zu 60 Gew.-%, bezogen auf Gemisch, 1-Isocyanato-3,3,5-trimethyl-5-isocyanatomethyl-cyclohexan und/oder 4,4'*-*Diisocyanatodicyclohexylmethan und/oder 1-Methyl-2,4(6)-diisocyanatocyclohexan mit
b) eine Diolkomponente, bestehend aus
   b1) mindestens einem Polyesterdiol eines aus dem Hydroxylgruppengehalt berechenbaren Molekulargewicht von 500 bis 10 000 aus (i) Adipinsäure und/oder Bernsteinsäure und (ii) mindestens einem Alkandiol mit 2 bis 6 Kohlenstoffatomen oder
   b2) einem Gemisch derartiger Polyesterdiole mit bis zu 32 Gew.-%, bezogen auf das Gesamtgewicht der Komponente b), an gegebenenfalls Ethergruppen aufweisenden Alkandiolen mit 2 bis 6 Kohlenstoffatomen,
c) eine Diaminkomponente in einer Menge von 2 bis 50 Äquivalent-%, bezogen auf die Gesamtmenge der in den Komponenten b) und c) vorliegenden, gegenüber Isocyanatgruppen reaktionsfähigen Gruppen, bestehend aus
   c1) Diaminosulfonaten der allgemeinen Formel

      H₂N-(-CH₂-)ₙ-NH-(-CH₂-)ₘ-SO₃Me

      oder
   c2) Gemischen aus Diaminosulfonaten c1) mit bis zu 70 Gew.-%, bezogen auf das Gesamtgewicht der Komponente c), an Ethylendiamin,
d) gegebenenfalls hydrophile Polyetheralkohole der allgemeinen Formel

   H-X-O-R

   in einer Menge von bis zu 10 Gew.-%, bezogen auf das Gesamtgewicht der Komponenten b), c) und d), sowie
e) gegebenenfalls Wasser, welches nicht in die Berechnung des Äquivalentverhältnisses von Isocyanatgruppen zu gegenüber Isocyanatgruppen reaktionsfähigen Gruppen eingeht,
umgesetzt, wobei in den genannten allgemeinen Formeln
- m und n: unabhängig voneinander für Zahlen von 2 bis 6 stehen,
- Me: für Kalium oder Natrium steht,
- R: für einen einwertigen Kohlenwasserstoffrest mit 1 bis 12 Kohlenstoffatomen steht, und
- X: eine Polyalkylenoxid-Kette des Molekulargewichtsbereichs 88 bis 4000 bedeutet, deren Alkylenoxideinheiten zumindest zu 40 % aus Ethylenoxideinheiten und zum Rest aus Propylenoxideinheiten bestehen.

Man erhält so wäßrige Dispersionen von Polyesterpolyurethanpolyharnstoffen.

Der benutzte Begriff "wäßrige Dispersion" soll auch wäßrige Lösungen umfassen, die dann vorliegen können, wenn die Konzentration an hydrophilen Zentren in den Harnstoffgruppen aufweisenden Polyurethanen ausreichend hoch ist, um eine Wasserlöslichkeit zu gewährleisten. Oftmals handelt es sich bei den Dispersionen um wäßrige Systeme, die sowohl dispergierte als auch gelöste Harnstoffgruppen aufweisende Polyurethane enthalten.

Zur Herstellung der wäßrigen Dispersionen werden die bereits obengenannten Ausgangsmaterialien a), b), c) und gegebenenfalls d) und/oder gegebenenfalls e) in den genannten Mengenverhältnissen eingesetzt.

Die Diisocyanatkomponente a) besteht vorzugsweise ausschließlich aus Hexamethylendiisocyanat oder einem Hexamethylendisocyanat gemisch mit insgesamt bis zu 60 Gew. % 1-Isocyanato-3,3,5-trimethyl-5-isocyanatomethylcyclohexan und/oder 4,4-Diisocyanatodicyclohexylmethan und/oder 1-Methyl-2,4(6)-diisocyanato-cyclohexan.

Die Diolkomponente b) besteht entweder aus b1) mindestens einem Polyesterdiol oder b2) aus einem Gemisch aus mindestens einem Polyesterdiol b1) mit bis zu 32, vorzugsweise bis zu 10 Gew.-% mindestens eines, gegebenenfalls Ethergruppen aufweisenden Alkandiols mit 2 bis 6 Kohlenstoffatomen.

Geeignete Polyesterdiole b1) sind solche eines aus dem Hydroxylgruppengehalt errechenbaren Molekulargewichts 500 bis 10 000, vorzugsweise 1 000 bis 2 500 auf Basis von (i) Adipinsäure und/oder Bernsteinsäure und (ii) gegebenenfalls Ethergruppen aufweisenden Alkandiolen mit 2 bis 6 Kohlenstoffatomen wie z.B. Ethylenglykol, Diethylenglykol, 1,4-Butandiol, Neopentylglykol und/oder 1,6-Hexandiol. Polyesterdiole, bei deren Herstellung ausschließlich Ethylenglykol und/oder 1,4-Butandiol als Diol eingesetzt worden sind, sind besonders bevorzugt.

Bei den gegebenenfalls als Hydroxylgruppen aufweisenden Kettenverlängerungsmitteln mitzuverwendenden, gegebenenfalls Ethergruppen aufweisenden Alkandiolen mit 2 bis 6 Kohlenstoffatomen handelt es sich um solche der soeben beispielhaft genannten Art.

Die Diaminkomponente c) besteht entweder aus c1) aus Diaminosulfonaten der bereits obengenannten allgemeinen Formel oder aus c2) Gemischen derartiger Diaminosulfonate mit Ethylendiamin, welches, falls überhaupt, in Mengen von bis zu 90, vorzugsweise bis zu 70 Äquivalent-%, bezogen auf die gegenüber Isocyanatgruppen reaktionsfähigen Aminogruppen der Komponente c) zum Einsatz gelangt. Ganz besonders bevorzugte Diaminosulfonate sind die Kalium- oder Natriumsalze der N-(2-Aminoethyl)-2-aminoethansulfonsäure.

Die Diaminkomponente c) wird im allgemeinen in einer Menge von 1 bis 10, vorzugsweise 2 bis 5 Gew.-%, bezogen auf das Gewicht der Komponente b) mitverwendet.

Bei der gegebenenfalls mitzuverwendenden Aufbaukomponente d) handelt es sich um hydrophile, einwertige Polyetheralkohole der allgemeinen Formel

H-X-O-R

in welcher
- R und X: die bereits obengenannte Bedeutung haben.

Bevorzugt sind solche derartige Polyetheralkohole, für welche
- R: für einen aliphatischen Kohlenwasserstoffrest mit 1 bis 4 Kohlenstoffatomen steht und
- X: für eine Polyalkylenoxidkette des Molekulargewichtsbereichs 500 bis 4 000 steht, in welcher mindestens 40, insbesondere mindestens 70 und besonders bevorzugt 100 % der vorliegenden Alkylenoxideinheiten, Ethylenoxideinheiten und die restlichen Alkylenoxideinheiten Propylenoxideinheiten darstellen.

Die Formgebung und die gleichzeitige Einbettung des biologischen Materials erfolgt durch eine ionisch induzierte Koazervation der Polyesterpolyurethanpolyharnstoff, bei der die Polysaccharidkomponente eingeschlossen wird. Das Verfahren zur Einbettung kann sowohl in einem Schritt, als auch in einem mehrstufigen Prozeß durchgeführt werden. Beim einstufigen Verfahren werden biologisches Material, Polyesterpolyurethanpolyharnstoff und biologisches Material gemeinsam vermischt und durch Zugabe zu einer wäßrigen Salzlösung zur Koazervation gebracht. Dieser Einschlussprozess wird wesentlich durch die Viskosität der verwendeten Polyesterpolyurethanpolyharnstoffe/Polysaccharid Mischung im verwendeten Lösungsmittel bestimmt.

In einem zweistufigen Verfahren können durch die Wahl eines geeigneten Polysaccharids wie z.B. Alginat zunächst Hydrogelkugeln erzeugt werden, die aus einem Polysaccharid bestehen. Diese Hydrogelteilchen können durch Eintauchen in eine wäßrige Lösung des Polyesterpolyurethanpolyharnstoffes mit einer mechanisch stabilen Hülle versehen werden.

Als erfindungsgemäße Polysaccharidkomponente des Hydrogels können sämtliche biologisch abbaubaren Polysaccharide oder deren Derivate einzeln oder in Mischung verwendet werden. Geeignet sind beispielsweise native und lösliche Stärke beliebiger Provenienz, Amylose, Amylopektin, Alginsäuren, Alginate, Carrageenan, Chitin, Chitosan, Dextran, Glycogen, Guar, Johannisbrotkernmehl, Laevan, Pektin, Pullulan, Tamaridenkernmehl, Xanthan und Hylan, sowie Cellulose beliebiger Provenienz. Geeignet sind ebenfalls Cellulosederivate, wie z.B. Celluloseether, Celluloseester und Cellulosecarbamate.

Besonders geeignet sind z.B. Celluloseether wie Methylcellulose, Ethylcellulose oder Benzylcellulose mit durchschnittlichen Substitutionsgraden kleiner oder gleich 2.5, Hydroxyethylcellulose, Hydroxypropylcellulose, Dihydroxypropylcellulose, Hydroxybutylcellulose, Methylhydroxyethylcellulose, Methylhydroxypropylcellulose, Methylhydroxybutylcellulose, Ethylhydroxypropylcellulose, Ethylhydroxyethylcellulose, Carboxyalkylcellulose, Sulfoalkylcellulose, Cyanoethylcellulose und deren Mischether. Besonders bevorzugt werden Methylcellulose, Hydroxyethylcellulose oder Hydroxypropylcellulose eingesetzt. Weiterhin geeignet sind Polysacchridderivate insbesondere Cellulosederivate mit beliebigen Mischungen aus Ether-, Ester- und Carbamatsubstituenten.

Die erfindungsgemäßen Polyurethan-Polysaccharid-Kombinationen, im folgenden auch als "blend" bezeichnet, lassen sich durch Autoklavieren sterilisieren und sind vollständig biologisch abbaubar.

Desweiteren ermöglichen sie die Kontrolle und Einstellung weiterer Eigenschaften, nämlich von Wasserhaushalt, Formstabilität, Permeabilität für Sauerstoff und Nährstoffe, Einstellung physiologischer Bedingungen, mechanischer Abbau z.B. durch auskeimende Pflanzen, sowie Einlagerung und Permeabilität für Nähr-, Schutz- und Wirkstoffe.

Es muß als überrraschend angesehen werden, daß die blends Kombinationen von Eigenschaften aufweisen, die für den Verwendungszweck, nämlich die Verkapselung von teilungsfähigem, biologischen Material von Vorteil sind:
- kann in wäßrigen Lösungsmitteln verarbeitet werden.
- kann bei physiologischen Temperaturen (18°-30°C) verarbeitet werden.
- kann durch Autoklavieren sterilisiert werden, ohne seine Eigenschaften zu verlieren.
- ist vollständig biologisch abbaubar und kompostierbar.
- kann in einfachen, wirtschaftlichen Verfahren zur Verkapselung eingesetzt werden
- ist für Pflanzen untoxisch
- kann so verarbeitet werden, daß Wasser und Gasaustausch gewährleistet sind
- führt zu befriedigenden Keimraten

Ein weiterer Gegenstand der Erfindung sind Wasser enthaltende Einbettungsmassen für biologisches Material, welche einen vollständig biologisch abbaubaren Polyesterpolyurethanpolyharnstoff und wenigstens ein vollständig biologisch abbaubares ionisches oder neutrales Polysaccharid oder Polysaccharidderivat enthalten.

Vorzugsweise besteht die Einbettungsmasse zumindestens zu 20 Gew. % aus dem vorstehend beschriebenen Polyesterpolyurethanpolyharnstoff und wenigstens zu 0.1 Gew. % aus einer Polysaccharidkomponente wie z.B. aus Stärke, einem Stärkederivat, Cellulose, einem Celluloseether oder beliebigen Mischungen solcher.

Bevorzugt sind hierbei in Wasser lösliche oder zumindest gut quellbare Polysacccharidderivate wie z.B. Stärke, Stärkeether oder Celluloseether sowie wäßrige 5- 50 Gew.% ige Dispersionen des Polyesterpolyurethanpolyharnstoffes. Besonders bevorzugt sind hierbei lösliche Stärke Alginate Methylcellulose, Hydroxyethylcellulose, Methylhydroxypropylcellulose, Methylhydroxyethylcellulose und/oder Hydroxypropylcellulose.

Ein weiterer Gegenstand der Erfindung ist ein Verfahren zur Einbettung von biologischem Material, bei dem man das biologische Material in Gegenwart einer wäßrigen Dispersion eines Polyesterpolyurethanpolyharnstoffes mit einem Polysaccharid und/oder Polysaccharid-Derivat vermischt und durch Kontaktieren mit einer wäßrigen Salzlösung diese Mischung zur Koazervation bringt. Durch diese ionisch induzierte Koazervation des Polyesterpolyurethanpolyharnstoffes wird die Polysaccharidkomponente und das beigefügte biologische Material eingeschlossen, wobei dieser Einschlussprozess wesentlich durch die Viskosität der verwendeten Polyesterpolyurethanpolyharnstoffe/Polysaccharid Mischung im verwendeten Lösungsmittel mitbestimmt wird.

Bevorzugt muß die kinematische Viskosität der durch Ionen zu vernetzenden Lösung größer als 1,1·10⁻⁶ m²/s sein.

Das Verfahren zur Einbettung kann sowohl in einem Schritt als auch in einem mehrstufigen Prozeß durchgeführt werden. Beim einstufigen Verfahren werden biologisches Material, Polyesterpolyurethanpolyharnstoff und Polysaccharidkomponente gemeinsam vermischt und durch Zugabe zu einer wäßrigen Salzlösung zur Koazervation gebracht. Es resultieren Hydrogelpartikel, die entsprechend dem Verfahren in Form von Kugeln, Schläuchen etc. hergestellt werden können. Das Hydrogel-Einbettungsmaterial besteht hierbei aus einem Blend von Polysaccharid und Polyesterpolyurethanpoiyharnstoff.

In einem zweistufigen Verfahren können durch die Wahl eines geeigneten Polysaccharids wie z.B. Alginat zunächst Hydrogelkugeln erzeugt werden, die aus einem Polysaccharid bestehen. Diese werden durch Eintropfen einer Mischung von Polysaccharid und biologischem Material in eine Salzlösung erhalten. Die Hydrogelteilchen enthalten noch ausreichende Mengen an Ionen für die Koazervation von Polyesterpolyurethanpolyharnstoff. Deshalb können diese Hydrogelteilchen durch Eintauchen in eine wäßrige Lösung des Polyurethanpolyharnstoffs mit einer mechanisch stabilen Hülle versehen werden.

Grundsätzlich bestehen also mindestens 2 Möglichkeiten um den Einbettungsprozeß des biologischen Material in Polysaccharid-Polyesterpolyurethanpolyharnstoff -Hydrogelen durchzuführen.

Generell kann dieser Prozeß durch Variation der Zusammenfügung von biologischem Material, Polysaccharid, Polyesterpolyurethanpolyharnstoff und Ionen variiert werden, wobei immer die Zusammenwirkung von Polyesterpolyurethanpolyharnstoff und Ionen zur Koazervation und somit zur Einbettung und Formgebung führt, so daß dieser Schritt zuletzt erfolgen muß, jedoch beliebige Mischungen A und B verwendet werden können, wobei Mischung A aus Polysaccharid, Polyesterpolyurethanpolyharnstoff und/oder biologischem Material bestehen kann und Mischung B aus Ionen, biologischem Material und Polysaccharid bestehen kann.

In einer besonders bevorzugten einstufigen Ausführungsform wird die Polysaccharidkomponente in einer wäßrigen Dispersion des Polyesterpolyurethanpolyharnstoffes aufgequollen oder gelöst, das biologische Material zugegeben und die resultierende Mischung durch Zusatz von Ionen, bevorzugt mehrwertigen Ionen wie insbesondere Ca²⁺, Mg²⁺, oder Al³⁺ in Form ihrer Chloride im Bereich von 10 - 1000 mM zur Koazervation gebracht, wobei durch diesen Vorgang eine Formgebung zu Kugeln, Fasern, Folien oder anderen Formkörpern erfolgen kann. Hierbei entstehen Hydrogele, die aus einem Blend von Polysaccharid und Polyesterpolyurethanpolyharnstoff bestehen.

In einer weiteren bevorzugten zweistufigen Ausführungsform wird das biologische Material mit Ionen und Polysaccharid in einem wäßrigen Lösungsmittel vermischt und durch Zugabe zu einer Polyurethanharnstoffdispersion eine Einbettung in einem Polysaccharidhydrogel vorgenommen, welches von einer Polyurethanpolyharnstoffhülle umhüllt ist.

Im Verfahren zur Einbettung können den Einbettungsmassen Nähr-, Schutz- und Wirkstoffe, die das Wachstum oder den Metabolismus des einzubettenden biologischen Materials begünstigen, sowie dieses vor schädlichen Einflüssen schützen, zugesetzt werden.

In einer bevorzugten Ausführungsform kann die Einbettungsmasse in halbkonzentriertem Nährmedium der Zusammensetzung nach Murashige und Skoog (veröffentlicht in Physiol. Plant. 15, 473, 1962) hergestellt werden, welchem 5-20g/l Saccharose, bevorzugt aber 10g/l Saccharose zugesetzt wurde.

Es können auch abhängig vom eingebetteten Pflanzenmaterial beliebige andere Nährsalzmischungen, die z.B. kommerziell erhältlich sind, und Zucker verwendet werden. Die Nährmedien können zur Beeinflussung der Entwicklung die dem Fachmann bekannten Phytohormone enthalten. Bei Nährstoffen handelt es sich um die abhängig vom Pflanzenmaterial gebräuchlichen und kommerziell erhältlichen Nährsalz- und Vitaminmischungen sowie um ggf. ebenfalls kommerziell erhältliche natürliche oder synthetische Phytohormone z.B. aus der Klasse der Auxine, Cytokinine, Gibbereline, Abscisinsäure, sowie ethylenbildende Substanzen. Desweiteren Verbindungen, die vitamin- oder phytohormonähnliche Wirkungen haben, wie z.B. Chlorocholinchlorid, Lipo-Oligosaccharide, Salicylsäurederivate.

In einer besonderen Ausführungsform können dem Einbettungsmaterial zum Schutz des sich teilenden Pflanzenmaterials bakterizide, fungizide, insektizide, akarizide, nematizide und bei entsprechender natürlicher oder gentechnisch erzeugter Toleranz auch herbizide Wirkstoffe zugesetzt werden. Bei Schutzstoffen handelt es sich z.B. um Insektizide, z.B. aus den Klassen der Phosphosäurester, Carbamate, insbesondere Imidacloprid, oder z.B. um Fungizide aus den Klassen der Azole, insbesondere Triadimenol und Tebuconazol.

Als Beispiele für Fungizide seien genannt:
2-Aminobutan; 2-Anilino-4-methyl-6-cyclopropyl-pyrimidin; 2',6'-Dibromo-2-methyl-4'-trifluoromethoxy-4'-trifluoromethyl-1,3-thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Dichlofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickeldimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram,
8-tert.-Butyl-2-(N-ethyl-N-n-propyl-amino)-methyl-1,4-di oxa-spiro-[4,5]decan,
N-(R)-(1-(4-Chlorphenyl)-ethyl)-2,2-dichlor-1-ethyl-3t-methyl-1r-cyclopropancarbonsäureamid (Diastereomerengemisch oder einzelne Isomere),
[2-Methyl-1-[[[1-(4-methylphenyl}-ethyl]-amino]-carbonyl]-propyl]-carbaminsäure-1-methylethylester und
1-Methyl-cyclohexyl-1-carbonsäure-(2,3-dichlor-4-hydroxy)-anilid.

Als Beispiele für Bakterizide seien genannt:
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Als Beispiele für Insektizide, Akarizide und Nematizide seien genannt:
Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton-M, Demeton-S, Demeton-S-methyl, Diafenthiuron, Diazinon, Dichlofenthion, Dichlorvos, Didiphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Als Schutzstoffe können außerdem chemische oder biologische Resistenzinduktoren Verwendung finden, die einen Schutz der Pflanze gegen phytopathogene Mikroorganismen wie Pilze, Bakterien, Viren oder Viroide bewirken. Manche Verbindungen mit resistenzinduzierender Wirkung bewirken einen Schutz gegen Insekten oder Nematoden. Beispiele für Stoffklassen mit resistenzinduzierender Wirkung sind Benzothiadiazole und ihre Derivate, Mono- und Dichlorisonicotinsäuren und ihre Derivate, Dichlorisothiazole und ihre Derivate, Dibromthiophencarbonsäuren und ihre Derivate, Salicylsäure und ihre Derivate sowie Probenazole. Bei den biologischen Resistenzinduktoren handelt es sich um Mikroorganismen, wie z.B. für die Pflanze nützliche Pilze, Bakterien oder Viren, die einen Schutz der Pflanze vor pathogenen Organismen, z.B. vor schädlichen Pilzen, Bakterien, Viren oder Nematoden bewirken.

Neben solchen Mikroorganismen können auch Organismen in den erfindungsgemäßen künstlichen Saatgut verwendet werden, die als Symbionten, wie z.B. als Mykorrhiza-Pilze, oder aber, wie z.B. Rhizobien im Zusammenhang mit der Stickstoff-Fixierung, das Wachstum von Pflanzen unterstützen. Auch durch die Bildung spezifischer Stoffwechselprodukte durch Mikroorganismen, die in Kombination mit dem pflanzlichen Material eingesetzt werden, kann die Auskeimung und das Wachstum der Pflanzen verbessert und die Pflanze gegen Pathogene und Schädlingsbefall geschützt werden.

Die erfindungsgemäßen Hydrogel-Einbettungsmassen können als Lager- oder Transportform für das biologische Material eingesetzt werden.

Ein weiterer Gegenstand der Erfindung ist weiterhin die Verwendung der so erhaltenen eingebetteten biologischen Materialien als künstliche Saatgut.

Die biologische Abbaubarkeit der erfindungsgemäßen Polyesterpolyurethanpolyharnstoffe sowie der Mischungen mit den erfindungsgemäßen Polysaccharidderivaten wurde wie unten beschrieben nachgewiesen. Die biologische Abbaubarkeit der aus den erfindungsgemäßen Materialien geformten Einbettungsmassen wurde ebenfalls in Kompost und Boden gezeigt. Nach spätestens 4 Wochen war das Material komplett abgebaut, ein Kontrollversuch mit biologisch nicht aktivem Substrat zeigte keinerlei Abbau, so daß eine Desintegration der Einbettungsmasse durch Hydrolyse oder mechanische Einflüsse ausgeschlossen werden kann. Der Abbau erfolgte auch in Gegenwart der erfindungsgemäß genannten Zusätze wie z.B. Wirkstoffe, Nährstoffe etc.

Die zu testenden Verbindungen werden in einem geeignetem Kasten in eine 2 cm hohe Mischung aus durchgerottetem Kompost aus einer Kompostieranlage, Rottegrad IV, eingelegt. Die gefüllten Kästen werden in einem Brutschrank für jeweils 4 Wochen nacheinander bei 60, 50 und 37°C inkubiert. Wasserverluste werden über den Gewichtsverlust bestimmt und ausgeglichen. Während der Inkubation wird regelmäßig der pH-Wert des Komposts gemessen. Wenn der gemessene Wert um mehr als eine Einheit von pH 7 abweicht, wird durch 100 mM Kalium-Phosphat pH 7,0 eingestellt. Nach jeweils 1 Woche wird ein Ansatz abgebrochen, die Materialien entnommen, gereinigt, bei 80°C bis zur Gewichtskonstanz getrocknet und fotografiert. Unmittelbar nach dem Trocknen wird der Gewichtsverlust des Materials durch erneutes Wiegen bestimmt.

In der vergifteten Kontrolle wird der Ansatz komplett bei 105°C getrocknet und das dabei verdampfte Wasser dann durch eine 0,1 %ige HgCl₂-Lösung ersetzt. Die Proben für die vergiftete Kontrolle werden vor dem Einbringen in das Kompostgemisch in die HgCl₂-Lösung eingelegt und dann getrocknet. Der Kontrollansatz wird genauso inkubiert wie die zu testenden Ansätze. Eine Substanz wird dann als abbaubar eingestuft, wenn nach 4 Wochen im unvergifteten Ansatz keine Probensubstanz mehr nachzuweisen, die Probe im vergifteten Ansatz jedoch unverändert ist.

Die Erfindung soll anhand der nachfolgenden Beispiele noch näher erläutert werden, ohne sie jedoch zu beschränken.

### Beispiele

In den Beispielen wird als Polyesterpolyurethanpolyharnstoff der Polyesterpolyurethanpolyharnstoff gemäß der DE 19 517 185 verwendet. Als Hydroxyethylcellulose bzw. Hydroxypropylcellulose werden in den Beispielen wasserlösliche, biologisch abbaubare Hydroxyalkylcelluloseether mit einem mittleren Molekulargewicht (Zahlenmittel) von ca. 10.000 bis 200.000 g/mol und einem Substitutionsgrad bezüglich der Ethergruppen von ca. 0,5 bis 1,5 verwendet.

### Beispiel 1

Die Kartoffelpflanzen (Solanum tuberosum) wurden in vitro vermehrt. Hierzu wurden Sproßabschnitte mit 2 bis 6 Blattachseln auf flüssiges BM-Medium mit 20 g/l Saccharose aufgelegt und bei einem Licht/Dunkel-Rhythmus von je 12 Stunden bei 22°C während der Tagesperiode und 19°C während der Nachtperiode im Pflanzenschrank inkubiert. Das BM-Medium besteht aus Salzen nach Murashige/Skoog (vgl. Murashige T., Skoog, Physiol. Plant. 15, 473-479, 1962) und Vitaminen entsprechend Gamborgs Medium B5 (Gamborg O.L., Miller R.A., Ojima K., Exp Cell Res 50, 151, 1968; Gamborg O.L., Murashige T., Thorpe T.A., Vasil I.K., In Vitro 12, 473, 1976). Nach 3 bis 4 Wochen wurden Sproßabschnitte aus diesen Pflanzen gewonnen und für Verkapselungsversuche eingesetzt.

Die Sproßabschnitte wurden unter sterilen Bedingungen in einer 3%igen Dispersion von Hydroxypropylcellulose (HPC; mit Zusatz von 0,2 M CaCl₂ in halbkonzentrierter Nährlösung nach Murashige-Skoog) suspendiert und unter Rühren in eine 1%ige Alginatlösung eingetropft. Anschließend wurden die Kugeln unter Rühren mit einer 0,2 M CaCl₂-Lösung gewaschen. Danach wurden die Kugeln unter leichtem Rühren in eine 5%ige wäßrige Dispersion von Polyesterpolyurethanpolyharnstoff eingebracht, wobei sich an der Kugeloberfläche eine dünne, elastische Hülle aus Polyesterpolyurethanpolyharnstoff ausbildete. Nach 5 Minuten wurden die Kugeln, die einen Durchmesser von ca. 5 mm besaßen, aus der Lösung entfernt und mit 0,2 M CaCl₂-Lösung gewaschen. Die Kugeln wurden zum Auskeimen auf Agarplatten mit halbkonzentriertem Nährmedium nach Murashige-Skoog ausgelegt. Die Inkubation erfolgte bei 20°C und täglich 12 Stunden Belichtung im Pflanzenschrank.

Nach ca. 2 bis 3 Wochen wuchsen kleine Pflanzen aus den Polymerkugeln. Die Keimrate betrug 66 %.

### Beispiel 2

Das zu verkapselnde biologische Material aus Kartoffelpflanzen (Anzucht vgl. Beispiel 1) wurde unter sterilen Bedingungen in einer 3%igen Lösung von Natriumalginat suspendiert. Die Suspension wurde in eine 0,2 M CaCl₂-Lösung eingetropft, wodurch es zur Bildung von Alginatkugeln kam. Nach 30 Minuten wurden die Kugeln abgesaugt und in eine leicht gerührte 5%ige wäßrige Polyesterpolyurethanpolyharnstoff-Dispersion gegeben. An der Oberfläche des Alginat-Hydrogels bildete sich eine dünne elastische Umhüllung aus Polyesterpolyurethanpolyharnstoff. Nach 5 Minuten wurden die Kugeln aus der Lösung entfernt und gegebenenfalls nochmals in einer 0,1 M CaCl₂-Lösung gewaschen. Zum Auskeimen wurden die Kugeln, die einen Durchmesser von ca. 5 mm besaßen, auf Agarplatten mit halbkonzentriertem Nährmedium nach Murashige-Skoog ausgelegt. Die Inkubation erfolgte, wie unter Beispiel 1 angegeben, im Pflanzenschrank.

### Beispiel 3

75 ml einer 40%igen Dispersion eines Polyesterpolyurethanpolyharnstoffes und 75 ml einer 2%igen Dispersion von Hydroxyethylcellulose werden jeweils einzeln bei einer Temperatur von 121 °C 20 Minuten lang autoklaviert und anschließend unter sterilen Bedingungen im Verhältnis 1:1 vermischt.

Die Sproßabschnitte der Kartoffel wurden unter sterilen Bedingungen auf die Oberfläche dieses Gemischs aus Hydroxyethylcellulose und Polyesterpolyurethanpolyharnstoffes aufgebracht und einzeln mit Hilfe einer Pipette angesaugt.

Anschließend werden die Sproßabschnitte mitsamt dem sie umgebenden Gemisch aus Hydroxyethylcellulose und Polyesterpolyurethanpolyharnstoffes in eine 0,2 M CaCl₂-Lösung eingetropft. Nach einer Verweilzeit von 10 Minuten wurden die Kugeln, die einen Durchmesser von ca. 5 mm besitzen, entnommen und auf Agar mit halbkonzentriertem MS-Medium ausgelegt. Die Inkubation erfolgte bei 20°C und täglich 12 Stunden Belichtung im Pflanzenschrank. Die Auskeimrate betrug 90 % innerhalb von 2 bis 3 Wochen.

### Beispiel 4

75 ml einer 40%igen Dispersion eines Polyesterpolyurethanpolyharnstoffes und 75 ml einer 2%igen Dispersion von Hydroxypropylcellulose wurden jeweils einzeln bei einer Temperatur von 121°C 20 Minuten lang autoklaviert und anschließend unter sterilen Bedingungen im Verhältnis 1:1 vermischt.

Die Kartoffelpflanzen wurden in vitro vermehrt (vgl. Beispiel 1). Nach 3 bis 4 Wochen wurden Sproßabschnitte aus diesen Pflanzen gewonnen und für Verkapselungsversuche eingesetzt. Die Sproßabschnitte wurden unter sterilen Bedingungen auf die Oberfläche des Gemischs aus Hydroxypropylcellulose und Polyesterpolyurethanpolyharnstoff aufgebracht und einzeln mit Hilfe einer Pipette angesaugt.

Anschließend wurden die Sproßabschnitte mitsamt dem sie umgebenden Gemisch aus Hydroxypropylcellulosse und Polyesterpolyurethanpolyharnstoffes in eine 0,2 M CaCl₂-Lösung eingetropft. Nach einer Verweilzeit von 10 Minuten wurden die Kugeln, die einen Durchmesser von ca. 5 mm besitzen, entnommen und auf Agar mit halbkonzentriertem MS-Medium ausgelegt. Die Inkubation erfolgte bei 20°C und täglich 12 Stunden Belichtung im Pflanzenschrank. Die Auskeimrate lag zwischen 90 und 100 % innerhalb von 2 bis 3 Wochen.

### Beispiel 5

Zellsuspension von Karotte (Daucus carota) wurden in 50 ml hormonhaltigem Murashige-Skoog-Medium (MS-Medium; vgl. Murashige T., Skoog, F., Physiol. Plant. 15, 473-479, 1962) bei 100 Umdrehungen pro Minute und 25°C auf einer Schüttelmaschine im Dunkeln inkubiert.

Nach 8 Tagen wurden 150 ml der Zellsuspension über Siebe der Maschenweite 500 µm, 75 µm und 30 µm gegeben. Die Zelifraktion 30 bis 75 µm wurde mit hormonfreiem Medium abgespült, durch Zentrifugation bei 100 g sedimentiert, zweimal mit hormonfreiem MS-Medium gewaschen und nach erneuter Zentrifugation in 20 ml hormonfreiem MS-Medium aufgenommen. Die Zellzahl betrug in der Regel 0,5 x 10⁴ bis 10⁵ Zellen/ml.

Diese Zellen wurden zur Induktion der Embryogenese eingesetzt. Die gesiebten, gewaschenen Zellen wurden, wie oben angegeben, auf der Schüttelmaschine weiterinkubiert; nach 2 und 5 Tagen erfolgte ein Medienwechsel, wobei die Zellen abzentrifugiert und in hormonfreiem MS-Medium resuspendiert. Danach wurde weitere 9 Tage inkubiert. Nach insgesamt 14 Tagen enthielt die Suspension 10 bis 100 Embryoide/ml.

Somatische Embryonen der Stadien "Torpedo" und "Cotelydonary" aus Karotte wurden auf die Oberfläche eines Gemischs aus Hydroxypropylcellulose und Polyesterpolyurethanpolyharnstoff aufgebracht. Die Embryonen wurden einzeln mit Hilfe einer Pipette angesaugt und mit dem sie umgebenden Polymergemisch in eine 0,2 M CaCl₂-Lösung eingetropft. Nach einer Verweilzeit von 10 Minuten wurden die Kugeln, die einen Durchmesser von ca. 5 mm besitzen, entnommen und auf Agar mit halbkonzentriertem MS-Medium ausgelegt. Die Inkubation erfolgte bei 20°C und täglich 12 Stunden Belichtung im Pflanzenschrank. Nach 2 Wochen waren 20 % der Kugeln ausgekeimt.

### Beispiel 6

Überprüfung der biologischen Abbaubarkeit der Verkapselungen.

Die aus den Beispielen 1-5 erhaltenen Verkapselungen wurden wie vorstehend beschrieben in einem Kompostierversuch auf ihre vollständige biologische Abbaubarkeit überprüft. Im Abstand von einigen Tage wurde der Abbau überprüft. Der Kontrollversuch in vegiftetem Kompost zeigt, daß mikrobieller Abbau stattfindet.

| **Beispiel** | **7 Tage** | **20 Tage** | **32 Tage** | **42 Tage** | **Kontrolle** |
|---|---|---|---|---|---|
| 1 | intakt | verfärbt | beginnender Abbau | abgebaut | intakt |
| 2 | intakt | verfärbt | beginnender Abbau | abgebaut | intakt |
| 3 | intakt | verfärbt | beginnender Abbau | abgebaut | intakt |
| 4 | intakt, verfärbt | verfärbt | beginnender Abbau | abgebaut | intakt |
| 5 | intakt, verfärbt | verfärbt | beginnender Abbau | abgebaut | intakt |

### Beispiel 7

75 ml einer 40%igen Dispersion eines Polyesterpolyurethanpolyharnstoffes und 75 ml einer 2%igen Dispersion von Hydroxypropylcellulose die zusätzlich 2% Imidacloprid enthält, wurden jeweils einzeln bei einer Temperatur von 121°C 20 Minuten lang autoklaviert und anschließend unter sterilen Bedingungen im Verhältnis 1:1 vermischt. Dieses Gemisch wurde in ein 0.2 M CaCl2 Lösung eingetropft.

Die resultierenden ca. 5mm großen Kugeln enthalten ca. 30 mg/g des Wirkstoffes.

### Beispiel 8

### Trocknung/Rehydratisierung

Die in den Beispiel 1 bis 5 hergestellten Kugeln wurden an der Raumluft 7 Tage getrocknet und gewogen. Nach 24 Stunden Lagerung in Wasser wurde eine Gewichtszunahme um 45 % festgestellt, die sich auch bei längerer Lagerung in Wasser nicht weiter vergrößerte.

### Beispiel 9

### Kombination mit Wirkstoffen

75 ml einer 40 %igen Dispersion eines Polyurethanpolyharnstoffs und 75 ml einer 2 %igen Dispersion von Hydroxypropylcellulose wurden jeweils einzeln bei einer Temperatur von 121°C 20 Minuten lang autoklaviert und anschließend unter sterilen Bedingungen im Verhältnis 1:1 vermischt.

Eine Lösung des Herbizids Imidacloprid (1 Mol/l in DMF) wurde über Membranfilter (Porenweite 0,2 µm) sterilfiltriert und anschließend mit sterilem Wasser 1:10 verdünnt. Die erhaltene Stammsuspension von Imidacloprid wurde dem Gemisch aus Polyurethanpolyharnstoff und Hydroxypropylcellulose zu einer Endkonzentration von 0,1 mMol/l zugesetzt. Eine sterile 0,2 M CaCl₂-Lösung enthielt Imidacloprid in gleicher Endkonzentration.

Die Sproßabschnitte der Kartoffel (vgl. Beispiel 1) wurden unter sterilen Bedingungen auf die Oberfläche des Gemischs aus Hydroxypropylcellulose und Polyurethanpolyharnstoff aufgebracht und einzeln mit Hilfe einer Pipette angesaugt.

Anschließend wurden die Sproßabschnitte mitsamt dem sie umgebenden Gemisch aus Hydroxypropylcellulose und Polyurethanpolyharnstoff in eine 0,2 M CaCl₂-Lösung eingetropft. Als Kontrollversuch wurden Sproßabschnitte der Kartoffel ohne Imidacloprid verkapselt. Nach einer Verweilzeit von 10 Minuten wurden die Kugeln entnommen und auf Agar mit halbkonzentriertem MS-Medium ausgelegt. Die Inkubation erfolgte bei 20°C und täglich 12 Stunden Belichtung und 70 % Luftfeuchte im Pflanzenschrank.

Die Auskeimrate betrug 64 % innerhalb von 4 Wochen; die Kontrolle ohne Imidacloprid lag bei 57 % Auskeimung.

## Patentansprüche

1. Vollständig abbaubares Hydrogel bestehend aus wenigstens einem Polyesterpolyurethanpolyharnstoff sowie Polysacchariden und/oder Polysaccharid-Derivaten, welches biologisches Material enthält.

2. Hydrogele gemäß Anspruch 1, **dadurch gekennzeichnet, daß** sie als biologisches Material teilungsfähiges Pflanzenmaterial, insbesondere Pflanzenmaterial aus der Gruppe Pflanzenzellen, Kallusgewebe, Protoplasten, Pflanzengewebe, Pflanzenorgane zygotische Embryonen, somatische Embryonen, Protocorm-Analoge enthalten.

3. Hydrogele gemäß Anspruch 2, **dadurch gekennzeichnet, daß** sie als Pflanzenorgane Adventivsprossen, Mikroknollen, Achselknospen, Apialknospen oder Sprößlinge enthalten.

4. Hydrogele gemäß Anspruch 1, **dadurch gekennzeichnet, daß** sie als biologisches Material teilungsfähiges Material aus transgenen Pflanzen enthalten.

5. Hydrogele gemäß einem der vorherigen Ansprüche, **dadurch gekennzeichnet, daß** sie einen Polyesterpolyurethanpolyharnstoff aus der Umsetzung einer Diisocyanatkomponente a) mit einer Diolkomponente b), einer Diaminkomponente c), gegebenenfalls hydrophilen Polyetheralkoholen d) gegebenenfalls in Gegenwart von Wasser e), welches nicht in die Berechnung des Äquivalentverhältnisses von Isocyanatgruppen zu gegenüber Isocyanatgruppen reaktionsfähigen Gruppen eingeht, enthalten.

6. Hydrogele gemäß Anspruch 5, **dadurch gekennzeichnet, daß** sie einen Polyester polyurethanpolyharnstoff aus einer Umsetzung bei der als Diisocyanatkomponente a) Hexamethylendiisocyanat oder ein Hexamethylendiisocyanat-Gemisch mit insgesamt bis zu 60 Gew.-% 1-Isocyanato-3,3,5-trimethyl-5-isocyanatomethyl-cyclohexan und/oder 4,4'-Disocyanatodicyclohexylmethan und/oder 1 Methyl-2,4(6)-diisocyanto-cyclohexan eingesetzt wurde, enthalten.

7. Hydrogele gemäß einem der vorherigen Ansprüche, **dadurch gekennzeichnet, daß** sie als Polysaccharide und/oder Polysaccharid-Derivate lösliche Stärke, Alginate, Methylcellulose, Hydroxyethylcellulose Methylhydroxypropylcellulose, Methylhydroxyethylcellulose und/oder Hydroxypropylcellulose enthalten.

8. Hydrogele gemäß einem der vorherigen Ansprüche, **dadurch gekennzeichnet, daß** sie zur Pflanzenaufzucht geeignete Nährsalzmischungen, bakterizide, fungizide, insektizide, akarizide, nematizide resistenzinduzierende und/oder herbizide Wirkstoffe enthalten.

9. Für biologisches Material geeignete Einbettungsmassen enthaltend einen Hydrogel gemäß Anspruchs 1.

10. Einbettungsmassen gemäß Anspruch 9, **dadurch gekennzeichnet, daß** die Einbettungsmassen eine wäßrige Dispersion des Polyesterpolyurethanpolyharnstoffes in einer Menge von 5 bis 50 Gew.-% und ein Polysaccharid und/oder Polysaccharid-Derivat in einer Menge von wenigstens 0,1 Gew.-% enthalten.

11. Verfahren zur Herstellung von in vollständig abbaubaren Hydrogelen eingebetteten biologischen Materials, **dadurch gekennzeichnet, daß** man das biologische Material in Gegenwart einer wäßrigen Dispersion eines Polyesterpolyurethanpolyharnstoffes mit einem Polysaccharid und/oder Polysaccharid-Derivat vermischt und durch Kontaktieren mit einer Salzlösung die Mischung zur Koazervation bringt.

12. Verfahren gemäß Anspruch 10, **dadurch gekennzeichnet, daß** man eine Salzlösung mehrwertiger Ionen einsetzt.

13. Verwendung der biologisches Material enthaltenden Hydrogele gemäß einem der Ansprüche 1 bis 8 als künstliches Saatgut.

## Claims

1. Completely biodegradable hydrogel comprising at least one polyester polyurethane polyurea as well as polysaccharides and/or polysaccharide derivatives, the hydrogel containing biological material.

2. Hydrogels according to claim 1, **characterised in that** they contain as biological material plant material capable of dividing, especially plant material from the group comprising plant cells, callus tissue, protoplasts, plant tissue, plant organs, zygotic embryos, somatic embryos, protocorm analogues.

3. Hydrogels according to claim 2, **characterised in that** they contain as plant organs adventitious shoots, micronodules, axillary buds, apical buds or scions.

4. Hydrogels according to claim 1, **characterised in that** they contain as biological material material capable of dividing from transgenic plants.

5. Hydrogels according to one of the preceding claims, **characterised in that** they contain a polyester polyurethane polyurea formed from the reaction of a diisocyanate component a) with a diol component b), a diamine component c), optionally hydrophilic polyether alcohols d) optionally in the presence of water e), which is not included in the calculation of the equivalent ratio of isocyanate groups to groups that are reactive towards isocyanate groups.

6. Hydrogels according to claim 5, **characterised in that** they contain a polyester polyurethane polyurea formed from a reaction in which hexamethylene diisocyanate or a hexamethylene diisocyanate mixture with a total of up to 60 wt.% of 1-isocyanato-3,3,5-trimethyl-5-isocyanatomethyl-cyclohexane and/or 4,4'-diisocyanatodicyclo-hexylmethane and/or 1-methyl-2,4(6)-diisocyanato-cyclohexane was used as diisocyanate component a).

7. Hydrogels according to one of the preceding claims, **characterised in that** they contain as polysaccharides and/or polysaccharide derivatives, soluble starch, alginates, methyl cellulose, hydroxyethyl cellulose, methylhydroxypropyl cellulose, methylhydroxyethyl cellulose and/or hydroxypropyl cellulose.

8. Hydrogels according to one of the preceding claims, **characterised in that** they contain for plant breeding suitable nutrient salt mixtures, bactericidal, fungicidal, insecticidal, acaricidal, nematicidal and resistant-inducing and/or herbicidal active constituents.

9. Embedding compositions suitable for biological material containing a hydrogel according to claim 1.

10. Embedding compositions according to claim 9, **characterised in that** the embedding compositions contain an aqueous dispersion of the polyester polyurethane polyurea in an amount of 5 to 50 wt.% and a polysaccharide and/or polysaccharide derivative in an amount of at least 0.1 wt.%.

11. Process for producing biological material embedded in completely biodegradable hydrogels, **characterised in that** the biological material is mixed in the presence of an aqueous dispersion of a polyester polyurethane polyurea with a polysaccharide and/or polysaccharide derivative, and the mixture is coacervated by contact with a salt solution.

12. Process according to claim 10, **characterised in that** a salt solution of polyvalent ions is used.

13. Use of the biological material containing hydrogels according to one of claims 1 to 8 as artificial seed.

## Revendications

1. Hydrogel complètement dégradable consistant en au moins un polyester-polyuréthanne-polyurée, ainsi qu'en des polysaccharides et/ou dérivés de polysaccharides, qui contient un matériau biologique.

2. Hydrogels suivant la revendication 1, **caractérisés en ce qu'**ils contiennent comme matériau biologique, un matériau végétal susceptible de clivage, en particulier un matériau végétal parmi le groupe des cellules végétales, des tissus de cal, des protoplastes, des tissus végétaux, des embryons zygotiques d'organes végétaux, des embryons somatiques, des analogues de protocorm.

3. Hydrogels suivant la revendication 2, **caractérisés en ce qu'**ils contiennent comme organes végétaux, des jeunes pousses d'adventice, des microtubercules, des boutons de rameaux, des boutons apicaux ou des jeunes rameaux.

4. Hydrogels suivant la revendication 1, **caractérisés en ce qu'**ils contiennent comme matériau biologique, des matériaux susceptibles de clivage provenant de plantes transgéniques.

5. Hydrogels suivant l'une quelconque des revendications précédentes, **caractérisés en ce qu'**ils contiennent un polyester-polyuréthanne-polyurée provenant de la réaction d'un composant diisocyanate a) avec un composant diol b), un composant diamine c), le cas échéant un polyétheralcool hydrophile d), le cas échéant en présence d'eau e), qui n'intervient pas dans le calcul des rapports d'équivalents des radicaux isocyanate au radicaux pouvant réagir avec les radicaux isocyanate.

6. Hydrogels suivant la revendication 5, **caractérisés en ce qu'**ils contiennent un polyester-polyuréthanne-polyurée provenant d'une réaction au cours de laquelle comme composant diisocyanate a), on met en oeuvre l'hexaméthylènediisocyanate ou un mélange d'hexaméthylènediisocyanates avec au total, jusqu'à 60% en poids de 1-isocyanato-3,3,5-triméthyl-5-isocyanatométhylcyclohexane et/ou de 4,4'-diisocyanatodicyclohexylméthane et/ou de 1-méthyl-2,4(6)-diisocyanatocyclohexane.

7. Hydrogels suivant l'une quelconque des revendications précédentes, **caractérisés en ce qu'**ils contiennent comme polysaccharide et/ou dérivé de polysaccharide, de l'amidon soluble, un alginate, la méthylcellulose, l'hydroxyéthylcellulose, la méthylhydroxypropylcellulose, la méthylhydroxyéthylcellulose et/ou l'hydroxypropylcellulose.

8. Hydrogels suivant l'une quelconque des revendications précédentes, **caractérisés en ce qu'**ils contiennent des mélanges de sels nutritifs appropriés pour la croissance des végétaux, des agents actifs induisant la résistance aux bactéricides, fongicides, insecticides, acaricides, nématicides et/ou des agents actifs herbicides.

9. Masses d'implantation appropriées pour des matériaux biologique, contenant un hydrogel suivant la revendication 1.

10. Masses d'implantation suivant la revendication 9, **caractérisées en ce que** les masses d'implantation contiennent une dispersion aqueuse du polyester-polyuréthanne-polyurée en une quantité allant de 5 à 50% en poids et un polysaccharide et/ou un dérivé de polysaccharide en une quantité d'au moins 0,1% en poids.

11. Procédé de préparation de matériaux biologiques implantés dans des hydrogels complètement dégradables, **caractérisé en ce que** l'on mélange le matériau biologique en présence d'une dispersion aqueuse d'un polyester-polyuréthanne-polyurée, avec un polysaccharide et/ou un dérivé de polysaccharide et on réalise une coacervation par mise en contact du mélange avec une solution saline.

12. Procédé suivant la revendication 10, **caractérisé en ce que** l'on met en oeuvre une solution saline d'ions polyvalents.

13. Utilisation des hydrogels contenant un matériau biologique suivant l'une quelconque des revendications 1 à 8, comme semence artificielle.
